# FASCICULE DE BREVET EUROPEEN

(11) **EP 3 620 212 B1**
(45) Date de publication et mention de la délivrance du brevet: **24.03.2021**
(21) Numéro de dépôt: 19195311.6
(22) Date de dépôt: 04.09.2019
(51) Int. Cl.: A61K 36/86, A61Q 19/00, A61P 17/10, A61K 8/9789, A61P 17/02

(54) **EXTRAIT DE VIOLETTE FERMENTÉ, PROCÉDÉ D'OBTENTION D'UN TEL EXTRAIT ET UTILISATION EN COSMÉTIQUE**
FERMENTIERTES VEILCHENEXTRAKT, HERSTELLUNGSVERFAHREN EINES SOLCHEN EXTRAKTS UND EINSATZ IN DER KOSMETIK
FERMENTED VIOLET EXTRACT, METHOD FOR OBTAINING SUCH AN EXTRACT AND USE IN COSMETICS

(30) Priorité: 05.09.2018 FR 1857951
(43) Date de publication de la demande: 11.03.2020
(73) Titulaire: Le Jardin d'Elen - Atelier de Création, 31500 Toulouse (FR)
(72) Inventeur: ROLLAN, Serge, 09100 PAMIERS (FR); VIÉ, Hélène, 31500 TOULOUSE (FR)
(74) Mandataire: Ipside

(56) Documents cités:
- US-A1- 2005 089 499
- US-A1- 2015 224 158
- NAOHIRO OSHIMA ET AL: "Collagenase inhibitors from", JOURNAL OF NATURAL MEDICINES, SPRINGER-VERLAG, TO, vol. 67, no. 1, 13 avril 2012 (2012-04-13) , pages 240-245, XP035150988, ISSN: 1861-0293, DOI: 10.1007/S11418-012-0665-8
- KIM KYOUNG-SOOK ET AL: "Purification and structure determination of gelatinase and collagenase inhibitors from Viola patrinii fermentation extracts", IMMUNOPHARMACOLOGY AND IMMUNOTOXICOLOGY, vol. 32, no. 4, décembre 2010 (2010-12), pages 614-616, XP009512897, ISSN: 0892-3973
- DATABASE GNPD [Online] MINTEL; 1 décembre 2017 (2017-12-01), unknown: "340 Serum, Maria Galland", XP002790986, Database accession no. 5339243

## Description

La présente invention s'inscrit dans le domaine du traitement de la peau, des muqueuses et/ou des cheveux des individus, notamment de l'amélioration des propriétés cosmétiques de la peau, des muqueuses et/ou des cheveux des individus, et en particulier de la lutte contre le vieillissement cutané.

Plus particulièrement, la présente invention concerne un extrait fermenté d'une partie aérienne de violette, notamment de feuilles, qui est particulièrement adapté pour une utilisation en tant que principe actif cosmétique, notamment pour lutter contre le vieillissement cutané. L'invention concerne également un procédé d'obtention d'un tel extrait fermenté, ainsi qu'une composition contenant un tel extrait de feuilles de violette fermenté, et un procédé de traitement cosmétique de la peau mettant en œuvre une telle composition. L'invention concerne en outre un tel extrait fermenté pour son utilisation pour le traitement de l'acné et/ou des cicatrices.

La violette, nom commun utilisé pour désigner le genre végétal *Viola,* est une plante de la famille des *Violaceae.*

Diverses espèces du genre *Viola* sont utilisées en médecine depuis des décennies pour lutter contre divers troubles de la santé. Il a ainsi notamment été identifié et mis à profit, dans le domaine médical, des effets antifongique, antibactérien, analgésique, anti-inflammatoire et diurétique de ces espèces.

Le document US 2015/0224158 décrit notamment des applications médicales diverses d'extraits végétaux, notamment de feuilles de violette, obtenus par fermentation des végétaux par des grains de kéfir. Parmi de telles applications médicales, on peut notamment citer l'utilisation des extraits végétaux ainsi fermentés en tant qu'antiseptique, antitussif, anti migraine, etc.

De manière générale, la technologie de fermentation consiste à mettre en œuvre des microorganismes, plus particulièrement des bactéries et/ou des levures, qui décomposent la matière organique pour former des produits de fermentation de structures et de propriétés différentes en fonction du type de microorganismes utilisés. Ces produits de fermentation peuvent trouver application dans de nombreux domaines.

Les présents inventeurs se sont intéressés aux propriétés des extraits fermentés de parties aériennes, notamment des feuilles, de violette, notamment de l'espèce *Viola alba* (Besser, 1809), et en particulier de la sous-espèce *Viola alba* subsp. *dehnhardtii* (Ten.) (W. Becker, 1902), communément nommée « violette de Toulouse ».

Ils ont découvert au cours de leurs travaux que, de manière tout à fait surprenante, un extrait de partie aérienne, en particulier de feuilles, de violette ayant été soumis à fermentation par un ferment particulier, présente des propriétés qui s'avèrent tout à fait avantageuses dans le domaine cosmétique, et en particulier dans le domaine de la lutte contre le vieillissement cutané, processus complexe se traduisant par l'apparition de rides, le relâchement des tissus cutanés et sous-cutanés, une perte de l'élasticité de la peau, etc.

La présente invention vise ainsi à proposer un extrait de violette qui soit utilisable en tant que principe actif cosmétique, notamment pour un usage par voie topique, et qui soit efficace pour le soin, la protection et/ou le traitement de la peau.

L'invention vise notamment à ce que ce principe actif cosmétique permette de répondre à un objectif majeur du domaine cosmétique, à savoir de proposer aux consommateurs une composition cosmétique permettant de prévenir et/ou ralentir efficacement le vieillissement cutané.

Ainsi, il est proposé selon la présente invention un extrait fermenté d'une partie aérienne de violette, utilisable notamment en tant que principe actif cosmétique. On entend dans la présente description, de manière classique en elle-même, par le terme violette, une plante du genre *Viola.*

Cet extrait fermenté d'une partie aérienne de violette est obtenu par un procédé d'obtention comprenant des étapes de :
- préparation d'un extrait liquide par extraction aqueuse de la partie aérienne de violette,
- préparation d'une solution dite de fermentation, par mélange de cet extrait liquide avec un sucre fermentescible,
- ensemencement de cette solution de fermentation avec un mélange de microorganismes contenant au moins une espèce de bactéries acétiques et au moins une espèce de levure, ce mélange étant essentiellement dépourvu de bactéries lactiques et de levures fermentant le lactose,
- incubation de la solution de fermentation ainsi ensemencée à une température inférieure ou égale à 30 °C, notamment comprise entre 25 et 30 °C, pendant au moins 10 jours, de préférence pendant au moins 20 jours,
- et récupération de l'extrait fermenté liquide ainsi obtenu.

Le mélange de microorganismes mis en œuvre selon l'invention, contenant au moins une espèce de bactéries acétiques et au moins une espèce de levure, sera désigné dans la présente description, pour plus de commodité, par le terme « ferment ».

Les bactéries acétiques sont définies ici de manière classique en elle-même, comme des bactéries dont le métabolisme aérobie produit une fermentation acétique par oxydoréduction. Ce métabolisme se manifeste spécifiquement par un cycle de conversion complet ou partiel de glucides, d'alcools primaires, de polyols ou d'aldéhydes, dont résulte en particulier de l'acide acétique.

Selon la présente invention, le ferment mis en œuvre est essentiellement dépourvu, c'est-à-dire qu'il n'en contient pas, ou seulement en infimes quantités, de :
- bactéries lactiques, c'est-à-dire de bactéries aptes à produire de l'acide lactique, notamment par fermentation des sucres, en particulier des bactéries de la famille des *Lactobacillaceae,* et notamment du genre *Lactobacillus*;
- et de levures fermentant le lactose, telles que des levures de l'espèce *Kluyveromyces marxianus.*

La partie aérienne de violette utilisée pour la préparation de l'extrait fermenté selon l'invention peut consister en des feuilles, des fleurs, des tiges, ou tout mélange de celles-ci.

Dans des modes de réalisation particulièrement préférés de l'invention, le procédé est mis en œuvre à partir d'une partie aérienne de violette contenant des feuilles de violette, et préférentiellement essentiellement constituée de feuilles de violette. On entend, par essentiellement constituée de feuilles de violette, que la partie aérienne sélectionnée selon l'invention contient uniquement des feuilles de violette, à l'exclusion de toute autre partie de la plante, sauf en quantités minimes, c'est-à-dire inférieure à 1 % en poids.

Les présents inventeurs ont notamment découvert que la fermentation des extraits aqueux de partie aérienne de violette, et tout particulièrement de feuilles de violette, par un tel ferment, s'avère bien plus efficace que lorsqu'elle est réalisée par des grains de kéfir, comme proposé par l'art antérieur. Cette performance se mesure notamment en termes de vitesse et de rendement de la fermentation par unité de ferment mis en œuvre.

L'extrait fermenté obtenu selon l'invention présente des propriétés particulièrement avantageuses pour une application dans le domaine cosmétique, en particulier pour prévenir ou ralentir les effets du vieillissement de la peau. Il a par exemple été découvert par les présents inventeurs que mis au contact des cellules de la peau, l'extrait de violette fermenté selon l'invention induit une augmentation de la production, par ces cellules, d'une des protéines connues pour présenter un effet antivieillissement cutané, le collagène.

L'extrait de violette fermenté selon l'invention peut en outre répondre à l'une ou plusieurs des caractéristiques décrites ci-après, mises en œuvre isolément ou en chacune des leurs combinaisons techniquement opérantes.

Le procédé d'obtention de l'extrait fermenté selon l'invention est de préférence mis en œuvre à partir de violette de l'espèce *Viola alba,* et préférentiellement de la sous-espèce *Viola alba* subsp. *dehnhardtii.* Comme exposé ci-avant, cette sous-espèce est communément désignée par l'expression « violette de Toulouse ». Les extraits fermentés conformément à la présente invention d'une partie aérienne, et tout particulièrement de feuilles, de cette plante particulière, présentent un effet anti-âge particulièrement important.

L'étape d'extraction aqueuse d'une partie aérienne de violette peut être réalisée à partir de toute partie fraîche, par exemple de feuilles fraîches. Elle est de préférence réalisée à partir d'une partie séchée, notamment de feuilles séchées. Elle peut en outre aussi bien être réalisée sur la partie entière ou coupée en morceaux, que sur une poudre obtenue par broyage.

Ainsi, le procédé d'obtention de l'extrait fermenté selon l'invention peut comprendre une étape initiale de réduction en poudre de la partie aérienne, notamment des feuilles, de violette.

L'étape d'extraction aqueuse du procédé d'obtention de l'extrait fermenté selon l'invention peut être réalisée à froid. Elle est de préférence réalisée à chaud.

Dans des modes de mise en œuvre particuliers de l'invention, l'extraction aqueuse est réalisée à une température comprise entre 50 et 95 °C, de préférence entre 80 et 95 °C, par exemple à une température de 85 °C ± 2 °C ou de 90 °C ± 2 °C.

L'extraction aqueuse peut être réalisée par tout méthode classique en elle-même connue de l'homme du métier, notamment par infusion, décoction, percolation ou encore macération. Elle est de préférence réalisée par décoction.

La durée de l'extraction aqueuse est en outre de préférence comprise entre 20 et 60 minutes, notamment entre 20 et 30 minutes.

Préférentiellement, la quantité de matière solide (partie aérienne de violette) utilisée est comprise entre 10 et 30 g par litre d'eau.

Dans des modes de mise en œuvre particuliers du procédé d'obtention de l'extrait fermenté selon l'invention, l'extraction aqueuse est réalisée à 85 °C ± 2 °C pendant 20 à 30 minutes, à partir de 10 à 30 g de poudre par litre d'eau, et de préférence par décoction.

A l'issue de l'étape d'extraction aqueuse, le milieu réactionnel est de préférence soumis à filtration, de sorte à séparer les matières insolubles dans l'eau de l'extrait liquide obtenu.

Le procédé d'obtention d'un extrait fermenté liquide selon l'invention comprend ensuite une étape de préparation d'une solution de fermentation. Cette préparation comprend le mélange, avec l'extrait liquide obtenu par extraction aqueuse, d'un ou plusieurs sucres fermentescibles, c'est-à-dire de sucre(s) pouvant être fermenté(s) par les microorganismes, pour former notamment de l'alcool.

A titre d'exemples de sucre fermentescible pouvant être mis en œuvre selon l'invention, on peut citer le glucose ou le saccharose. Le saccharose, disaccharide constitué par la condensation de deux oses, une molécule de glucose et une molécule de fructose, est particulièrement préféré dans le cadre de l'invention. Des produits complexes contenant un ou des sucres fermentescibles, tels que le miel ou le sirop d'Agave, peuvent également être utilisé dans le cadre de l'invention.

La quantité de sucre fermentescible mélangé à l'extrait liquide pour former la solution de fermentation est par exemple comprise entre 50 et 70 g/l.

Dans des modes de mise en œuvre particulièrement avantageux du procédé d'obtention d'un extrait fermenté selon l'invention, le mélange de microorganismes (ferment) mis en œuvre contient :
- au moins une, de préférence plusieurs, espèces de bactéries acétiques, en particulier au moins une, de préférence plusieurs, espèces de bactéries du genre *Acetobacter,* par exemple *Acetobacter sp,* et/ou du genre *Gluconobacter,* par exemple *Gluconobacter oxydans* ;
- et au moins une, de préférence plusieurs, espèces de levure, en particulier au moins une, de préférence plusieurs, espèces de levures de la famille des *Saccharomycetaceae,* notamment du genre *Saccharomyces,* par exemple *Saccharomyces cerevisiae,* ou du genre *Zygosaccharomyces,* par exemple *Zygosaccharomyces sp,* et/ou de la famille des *Schizosaccharomycetaceae,* notamment du genre *Schizosaccharomyces,* par exemple *Schizosaccharomyces pombe.*

Pour l'ensemencement, il peut être utilisé différentes quantités de chacun des microorganismes entrant dans la composition du ferment, à tout rapport pondéral des uns par rapport aux autres.

Les bactéries sont de préférence utilisées dans une quantité comprise entre 10² et 10⁸ UFC (Unités Formant Colonies)/ml de solution de fermentation, de préférence comprise entre 10⁴ et 10⁷ UFC/ml.

Les levures sont quant à elles de préférence utilisées dans une quantité comprise entre 10² et 10⁷ UFC/ml de solution de fermentation, de préférence comprise entre 10³ et 10⁶ UFC/ml.

Les microorganismes peuvent être incorporés dans l'extrait aqueux isolément, ou bien sous forme de ferment symbiotique, c'est-à-dire d'un mélange de microorganismes s'étant naturellement développés conjointement en symbiose, notamment lors d'une culture de microorganismes en présence d'un extrait aqueux de la partie aérienne de violette et d'un sucre fermentescible, par exemple pendant au moins 15 jours à une température d'environ 25 °C.

Dans des modes de mise en œuvre particuliers du procédé d'obtention d'un extrait fermenté selon l'invention, le mélange de microorganismes est supporté sur un support solide, de préférence insoluble dans l'eau, notamment sur un support solide naturel produit par des microorganismes, et plus particulièrement par un ou plusieurs des microorganismes entrant dans la composition du ferment mis en œuvre selon l'invention.

Préférentiellement, le mélange de microorganismes est supporté sur un support solide gélatineux insoluble dans l'eau à base de cellulose, d'hémicellulose ou d'un de leurs dérivés, notamment à base d'hémicellulose bactérienne, en particulier produite par les bactéries de l'espèce *Gluconobacter oxydans.*

A titre d'exemple, l'hémicellulose formant le support solide peut répondre à la formule générale :

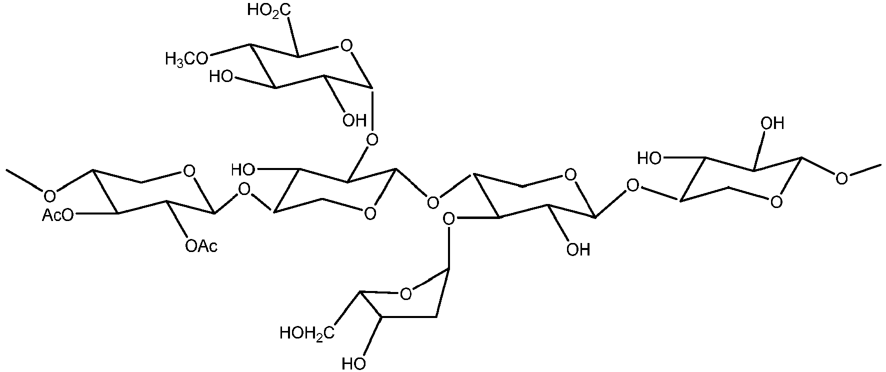

Le terme cellulosane est utilisé pour désigner cette hémicellulose.

L'incubation de la solution de fermentation ensemencée par le mélange de microorganismes selon l'invention est de préférence réalisée à une température comprise entre 25 et 30 °C.

Elle est en outre préférentiellement réalisée pendant une durée comprise entre 20 et 30 jours.

A l'issue de cette étape d'incubation, l'extrait fermenté liquide obtenu est récupéré. Cette récupération peut notamment être réalisée par filtration, de sorte à éliminer les résidus solides contenus dans le milieu liquide. La filtration peut notamment être réalisée sur un filtre de diamètres de pores d'1 µm.

Le cas échéant, de la glycérine peut être ajoutée au filtrat ainsi obtenu.

Dans des modes de mise en œuvre particuliers de l'invention, le procédé d'obtention d'un extrait fermenté liquide selon l'invention comprend une étape finale de stérilisation de l'extrait fermenté liquide récupéré à l'issue de l'incubation, notamment en conditions modérées, par exemple par tyndallisation.

A titre d'exemple, l'extrait fermenté liquide peut être soumis à tyndallisation par chauffage discontinu à 70 °C pendant une trentaine de minutes, à quelques heures d'intervalle, et ce à trois reprises. Un tel traitement permet avantageusement d'éliminer toutes les spores pouvant être contenues dans l'extrait fermenté liquide.

L'extrait fermenté liquide d'une partie aérienne de violette selon l'invention présente de préférence un pH inférieur ou égal à 3. Il présente en outre de préférence un degré Brix inférieur ou égal à 2, notamment compris entre 1 et 2. Il s'agit préférentiellement d'un extrait fermenté de feuilles de violette.

Le procédé d'obtention d'un extrait fermenté d'une partie aérienne de violette selon l'invention peut optionnellement comprendre une étape finale de lyophilisation de l'extrait, pour former un extrait fermenté en poudre.

Selon un autre aspect, la présente invention concerne un procédé d'obtention d'un extrait fermenté de partie aérienne de violette selon la présente invention. Ce procédé comprend des étapes de :
- préparation d'un extrait liquide par extraction aqueuse de la partie aérienne, notamment de feuilles, de violette,
- préparation d'une solution dite de fermentation par mélange de cet extrait liquide avec un sucre fermentescible,
- ensemencement de cette solution de fermentation avec un mélange de microorganismes contenant au moins une espèce de bactéries acétiques et au moins une espèce de levure, ce mélange étant essentiellement dépourvu de bactéries lactiques et de levures fermentant le lactose,
- incubation de la solution de fermentation ainsi ensemencée à une température inférieure ou égale à 30 °C pendant au moins 10 jours, de préférence pendant au moins 20 jours,
- et récupération de l'extrait fermenté liquide obtenu.

Ce procédé peut répondre à l'une ou plusieurs des caractéristiques décrites ci-avant en référence à l'extrait fermenté d'une partie aérienne de violette selon la présente invention.

Un autre aspect de la présente invention concerne une composition, en particulier une composition cosmétique, notamment applicable par voie topique sur la peau, les muqueuses et/ou les cheveux d'un individu. Cette composition contient un extrait fermenté d'une partie aérienne, en particulier de feuilles, de violette selon l'invention, répondant à l'une ou plusieurs des caractéristiques décrites ci-avant, dans un véhicule physiologiquement compatible.

On entend, par véhicule physiologiquement compatible, un véhicule adapté à une utilisation au contact de cellules humaines et animales, en particulier de cellules de la peau, notamment par application par voie topique. De préférence, ce véhicule présente une odeur, une couleur et un toucher agréables, et il ne génère pas d'inconforts inacceptables susceptibles de détourner un utilisateur de la composition selon l'invention.

La teneur en l'extrait fermenté d'une partie aérienne de violette selon l'invention dans la composition peut notamment être comprise entre 0,1 et 10 % en poids, par rapport au poids total de la composition.

Outre l'extrait fermenté d'une partie aérienne de violette selon l'invention, tel qu'il a été décrit ci-avant, la composition selon l'invention peut contenir un ou plusieurs ingrédient(s) actif(s) supplémentaires, notamment des ingrédients à propriétés bénéfiques pour la peau, et dont l'effet peut le cas échéant s'exercer en synergie avec celui de l'extrait de violette fermenté selon l'invention. A titre d'exemples, on peut notamment citer les agents hydratants, les agents émollients, les agents antirides, les agents antiradicalaires, les agents anti-UV, etc.

La composition selon l'invention peut également comporter un ou plusieurs additifs classiques en eux-mêmes, notamment dans le domaine cosmétique, par exemple un ou des parfum(s), agent(s) stabilisateur(s), agent(s) conservateur(s), émulsifiant(s), etc.

Cette composition peut se présenter sous toute forme classique en elle-même, notamment pour des compositions à usage topique, en particulier cutané, dans le domaine cosmétique, notamment sous forme d'une crème, d'une pommade, d'un lait, d'une lotion, d'un gel, d'un sérum, d'un onguent, etc.

Un autre aspect de l'invention concerne un procédé de traitement cosmétique non-thérapeutique de la peau, des muqueuses et/ou des cheveux d'un individu, notamment humain, ce procédé comprenant l'administration à cet individu, notamment par application par voie topique sur la peau, les muqueuses et/ou les cheveux de cet individu, d'une composition selon l'invention, répondant à l'une ou plusieurs des caractéristiques décrites ci-avant.

Le procédé de traitement cosmétique selon l'invention comprend notamment l'application de la composition selon l'invention par voie topique sur une zone saine, non atteinte par une maladie, en particulier par une maladie inflammatoire, de la peau, des muqueuses et/ou des cheveux de l'individu.

Le procédé de traitement cosmétique selon l'invention peut notamment comprendre l'application par voie topique, sur la surface cutanée des parties concernées du corps de l'individu en ayant besoin, par exemple sur la peau du visage et du cou, d'une quantité déterminée de la composition selon l'invention.

Cette application peut par exemple être réalisée à raison d'une ou deux fois par jour, par exemple le matin et le soir, et par exemple pendant une période comprise entre 2 semaines et 2 mois ou plus.

Appliquée par voie topique sur la peau, la composition selon l'invention permet notamment avantageusement de prévenir, ou du moins ralentir, l'apparition des signes cutanés du vieillissement.

Le procédé de traitement cosmétique selon l'invention peut autrement comprendre l'administration par voie orale, à l'individu concerné, de la composition selon l'invention.

Un autre aspect de l'invention concerne l'utilisation d'un fermenté extrait d'une partie aérienne, notamment de feuilles, de violette selon l'invention, répondant à l'une ou plusieurs des caractéristiques ci-avant, pour le traitement cosmétique non-thérapeutique de la peau, des muqueuses et/ou des cheveux d'un individu, en particulier pour lutter contre le vieillissement cutané.

Plus généralement, l'invention concerne l'utilisation d'un extrait d'une partie aérienne, notamment de feuilles, de violette, en particulier de l'espèce *Viola alba,* et préférentiellement de la sous-espèce *Viola alba* subsp. *dehnhardtii,* pour le traitement cosmétique non-thérapeutique de la peau, des muqueuses et/ou des cheveux d'un individu, en particulier pour lutter contre le vieillissement cutané

Cette utilisation peut notamment comprendre l'application de l'extrait de violette par voie topique, en particulier sur une zone de la peau de l'individu en ayant besoin, cette zone étant saine et non atteinte par une maladie, en particulier par une maladie inflammatoire.

Selon un autre aspect, la présente invention concerne un extrait fermenté d'une partie aérienne de violette selon l'invention pour son utilisation pour le traitement thérapeutique de désordres cutanés, en particulier à composante inflammatoire, notamment pour le traitement de l'acné et/ou des cicatrices, en particulier par application par voie topique sur la zone de peau affectée d'un individu, notamment humain. Cette application tire avantageusement profit de l'effet anti-inflammatoire de l'extrait fermenté selon l'invention.

En particulier, concernant le traitement de l'acné, il a été découvert par les présents inventeurs que, de manière tout à fait surprenante, l'extrait fermenté d'une partie aérienne de violette selon l'invention a la capacité de freiner l'apparition sur la peau de biofilms des bactéries *Propionibacterium acnes* et *Staphylococcus epidermitis,* qui sont impliqués dans des affections cutanées telles que l'acné (Burkart et Burkart, 2003, Int. J. Dermatol. 42: 925-927), et de bloquer la maturation de tels biofilms. Une telle action de l'extrait fermenté selon l'invention, qui a pour effet de favoriser la disparition des lésions acnéiques au niveau des peaux acnéiques et des peaux grasses à tendance acnéique, semble être due à une désactivation du quorum sensing, système de communication intercellulaire de ces bactéries.

De manière tout à fait avantageuse, l'extrait fermenté selon l'invention n'induit en outre pas d'intolérance, d'allergie ou d'effets secondaires agressifs au contact de la peau.

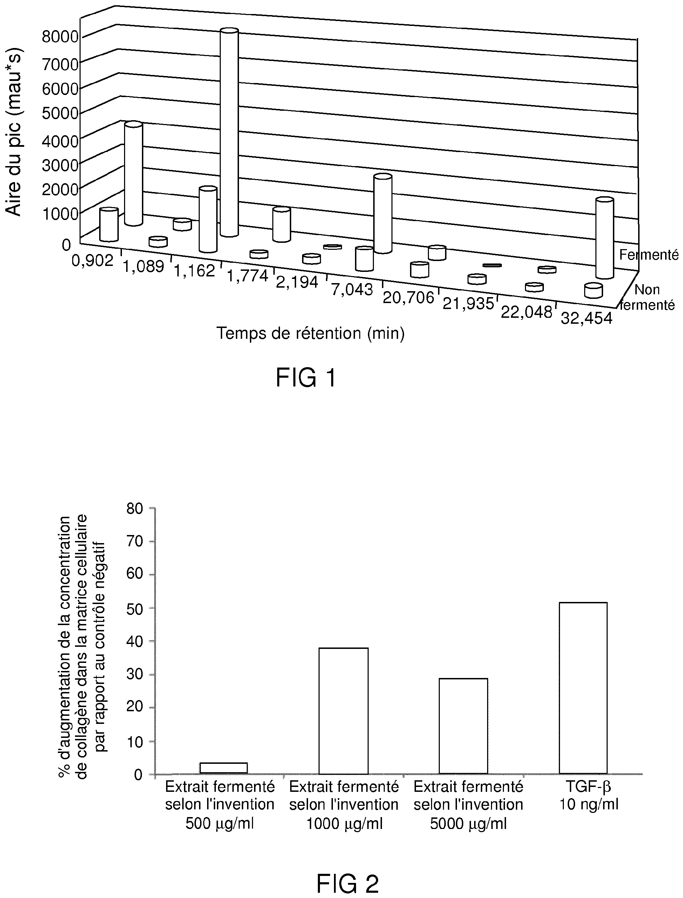

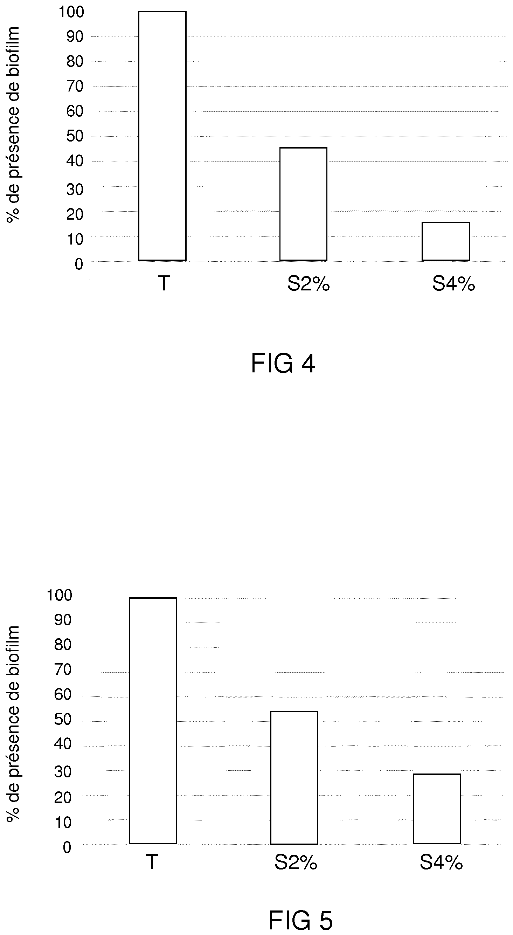

Les caractéristiques et avantages de l'invention apparaîtront plus clairement à la lumière des exemples de mise en œuvre ci-après, fournis à simple titre illustratif et nullement limitatifs de l'invention, avec l'appui des figures 1 et 5, dans lesquelles :
- la figure 1 représente des profils obtenus par analyse HPLC respectivement pour une solution de fermentation (non ensemencée) obtenue en produit intermédiaire lors de la mise en œuvre d'un procédé d'obtention d'un extrait fermenté de feuilles de *Viola alba* subsp. *dehnhardtii* selon l'invention (ligne avant), et pour un extrait fermenté de feuilles de *Viola alba* subsp. *dehnhardtii* selon l'invention, obtenu après fermentation de ladite solution de fermentation (ligne arrière), montrant uniquement les pics dont l'aire brute diffère entre les deux solutions ci-avant ;
- la figure 2 représente un graphe montrant le pourcentage d'augmentation de la quantité de collagène dans la matrice cellulaire de cellules de fibroblastes humains, induite par un extrait fermenté de feuilles de *Viola alba* subsp. *dehnhardtii* selon l'invention à différentes concentrations dans le milieu de culture des cellules, et par le facteur de croissance TGF-β à titre de contrôle positif, ce pourcentage étant calculé par rapport au milieu de culture non additionné ;

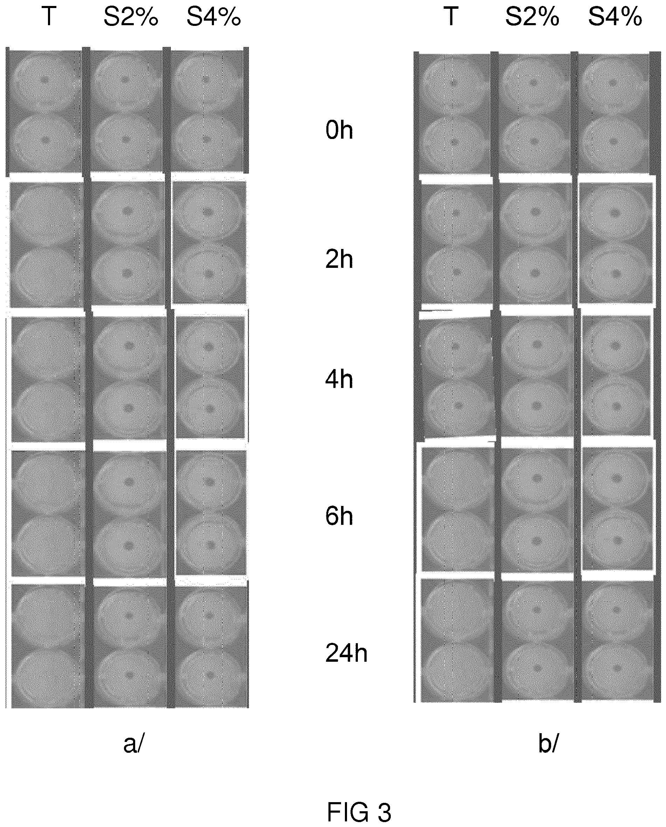

- la figure 3 montre des photographies de puits obtenus à l'issue de la mise en œuvre du test Biofilm Ring Test® pour un témoin (« T ») et pour des compositions contenant différentes concentrations d'un extrait fermenté selon l'invention (« S2% » et « S4% »), vis-à-vis de bactéries respectivement *Propionibacterium acnes* (a/) et *Staphylococcus epidermitis* (b/) après des temps d'incubation respectivement de 0h, 2h, 4h, 6h et 24h ;
- la figure 4 montre un graphe en barres représentant le pourcentage de présence de biofilm, calculé par le test Biofilm Ring Test®, pour un témoin (« T ») et pour des compositions contenant différentes concentrations d'un extrait fermenté selon l'invention (« S2% » et « S4% »), vis-à-vis de la bactérie *Propionibacterium acnes* après un temps d'incubation de 24h ;
- et la figure 5 montre un graphe en barres représentant le pourcentage de présence de biofilm, calculé par le test Biofilm Ring Test®, pour un témoin (« T ») et pour des compositions contenant différentes concentrations d'un extrait fermenté selon l'invention (« S2% » et « S4% »), vis-à-vis de la bactérie *Staphylococcus epidermitis* après un temps d'incubation de 24h.

### A/ Extrait fermenté de feuilles de Viola alba subsp. dehnhardtii

Il est mis en œuvre le procédé d'obtention conforme à l'invention suivant.

Des feuilles de l'espèce *Viola alba* subsp. *dehnhardtii* sont séchées, puis soumises à extraction aqueuse par décoction.

A cet effet, les feuilles séchées sont incorporées dans 400 l d'eau déminéralisée à température ambiante, à raison de 20 g de feuilles par litre d'eau. Le milieu aqueux est chauffé jusqu'à 85 °C ± 2 °C, et maintenu à cette température pendant 60 min, jusqu'à obtenir une densité optique supérieure à 20 à 280 nm.

Le milieu aqueux est ensuite filtré pour éliminer les résidus solides.

Une solution de fermentation est préparée à partir du filtrat ainsi obtenu.

Pour cela, du saccharose est ajouté dans le filtrat à une concentration de 70 g/l. Le milieu est agité jusqu'à dissolution complète du saccharose, puis filtré pour éliminer les impuretés solides restantes.

La solution de fermentation ainsi obtenue est ensemencée par un mélange de microorganismes contenant des bactéries des espèces *Acetobacter sp* et *Gluconobacter oxydans,* et des levures des espèces *Saccharomyces cerevisiae, Zygosaccharomyces sp* et *Schizosaccharomyces pombe,* ce mélange étant essentiellement dépourvu de bactéries lactiques et de levures fermentant le lactose.

Les microorganismes sont de préférence entrappés dans un support solide insoluble dans l'eau, de préférence à base d'hémicellulose.

Chaque microorganisme est mis en œuvre à raison d'environ 10⁶ UFC/ml de solution de fermentation.

Préférentiellement, il est mis en œuvre un ferment symbiotique qui a été obtenu par ensemencement d'une solution de fermentation contenant un extrait aqueux de feuilles de l'espèce *Viola alba* subsp. *dehnhardtii* et de saccharose, telle que la solution de fermentation décrite ci-avant, avec un mélange contenant un grand nombre de microorganismes différents, et incubation à environ 25 °C pendant 15 jours. Après 15 jours d'incubation, il s'est formé dans la solution un produit polymère, le cellulosane, qui emprisonne un mélange de microorganismes particulier, contenant des bactéries des espèces *Acetobacter sp* et *Gluconobacter oxydans* et des levures des espèces *Saccharomyces cerevisiae, Zygosaccharomyces sp* et *Schizosaccharomyces pombe,* ce mélange étant essentiellement dépourvu de bactéries lactiques et de levures fermentant le lactose. Après filtration, on récupère ce ferment symbiotique sur support solide.

20 kg de ce ferment sont incorporés dans la solution de fermentation. Le milieu ainsi obtenu est ensuite incubé en conditions statiques à 28 °C (± 2 °C) pendant 30 jours.

On obtient alors pour le milieu réactionnel un degré Brix inférieur à 2 et un pH inférieur ou égal à 3.

Ce milieu est soumis à filtration de sorte à séparer le ferment sur support solide de l'extrait fermenté liquide, tout d'abord sur un premier filtre de diamètre de pores de 1 µm, puis sur un deuxième filtre de diamètre de pores de 0,2 µm.

L'extrait fermenté liquide ainsi isolé est soumis à un traitement par tyndallisation, par répétition à 3 reprises du cycle suivant : chauffage à 70 °C pendant 20 min, refroidissement à température ambiante pendant 24 h.

L'extrait de feuilles de violettes fermenté alors obtenu est stocké à l'abri de la lumière.

Cet extrait de feuilles de violettes fermenté, obtenu conformément à la présente invention, est analysé par chromatographie liquide haute performance (HPLC). A titre comparatif, il est également analysé la solution de fermentation formée au cours du procédé d'obtention ci-dessus, non ensemencée par le ferment.

L'analyse HPLC est réalisée sur une colonne Agilent Poroshell 120 SB-C18, avec injection de 10 µl de chaque échantillon (ayant au préalable été concentré 3 fois par évaporation sous vide), à une température de 30 °C et un débit de 0,8 ml/min, avec détection à 210 nm. L'éluant A (eau et acide formique 7,5 mM) et l'éluant B (acétonitrile) sont utilisés en mode gradient, comme suit :
0 min A 95 % B 5 % ; 3 min A 95% B 5%, ; 10 min A 92% B 8% ; 12 min A 90% B 10% ; 17 min A 87% B13 % ; 20 min A 80% B 20% ; 27 min A 70% B 30 % ; 30 min A 50% B 50% ; 31 min A 0% B100% ; 34 min A 0% B 100% ; 35 min A 95% B 5% ; 38 min A 95% B 5%.

Les résultats obtenus sont montrés sur la figure 1. Sur cette figure, la ligne avant montre les résultats obtenus pour la solution liquide avant fermentation, et la ligne arrière montre les résultats obtenus pour la solution liquide après fermentation.

On observe clairement une modification importante de la composition de la solution due à la fermentation.

### B/ Extraits fermenté de feuilles d'autres espèces de Viola

Le procédé décrit dans l'exemple A/ ci-avant est mis en œuvre à partir de feuilles d'autres espèces de violettes :
- *Viola tricolor* (communément nommée pensée sauvage),
- *Viola odorata* (communément nommée violette odorante).

Pour chacune de ces espèces, on obtient un extrait fermenté conforme à l'invention.

### C/ Activation de la synthèse de collagène par des fibroblastes humains

L'extrait de feuilles de *Viola alba* subsp. *dehnhardtii* fermenté obtenu à l'exemple A/ est testé pour sa capacité à activer la synthèse de collagène par des fibroblastes humains.

A cet effet, des cellules de fibroblaste humain primaires sont cultivées dans un milieu de culture adapté (milieu DMEM 4,5 g/l glucose, 2 mM de L-glutamine, 10 % de sérum de veau fœtal (SVF) inactivé à la chaleur, 50 Ul/ml de pénicilline, 50 µg/l de streptomycine), en présence de différentes concentrations de l'extrait fermenté selon l'invention (5000 µg/ml, 1000 µg/ml, 500 µg/ml). La culture est réalisée à 37 °C sous atmosphère 5 % CO₂.

A titre de contrôle négatif, il est testé l'effet du milieu de culture seul, c'est-à-dire non additionné de l'extrait fermenté selon l'invention, à 1 % de SVF. A titre de contrôle positif, il est testé le facteur de croissance TGF-β à 10 ng/ml dans le milieu de culture à 1 % de SVF.

Pour chaque échantillon testé, la quantité de collagène présente dans la matrice cellulaire et dans le milieu de culture est déterminée après 48 h de culture, au moyen de colorant rouge Sirius (Direct Red 80), qui présente une affinité particulière pour la structure triple-hélice (Gly-X-Y)ₙ du collagène natif. La quantité totale de protéines en présence est également mesurée par le test de Bradford.

A cet effet, après 48 h de culture, le milieu de culture est prélevé et la couche de cellules est rincée avec 500 µl de cocktail inhibiteur de protéase. La couche de cellules est ensuite récupérée par grattage dans 500 µl de cocktail inhibiteur de protéase, puis les puits de culture sont rincés avec 500 µl du cocktail inhibiteur de protéase. Les deux volumes, qui constituent la matrice extracellulaire, sont rassemblés et traités par ultrasons pendant 40 s.

Le collagène est complexé avec le colorant rouge Sirius. Après centrifugation et lavage à l'acide chlorhydrique 0,1 M, le culot est solubilisé dans une solution d'hydroxyde de sodium à 0,5 M. L'absorbance du complexe colorant - collagène est mesurée à 540 nm. Une gamme d'étalonnage est établie entre 0 et 50 µg par tube de collagène.

La quantité totale de protéines en présence est également mesurée par le test de Bradford, selon le protocole décrit dans la publication de Bradford et al., 1976, Anal. Biochem. 72:248-54, contre une gamme d'étalonnage établie à partir d'une solution de BSA (albumine de sérum bovin) à 400 µg/ml dans du PBS (tampon phosphate salin).

Pour chaque échantillon, il est calculé le rapport en poids de collagène / protéines mesurées par le test de Bradford (dans la matrice cellulaire et dans le milieu de culture), ainsi que le % d'augmentation de la production de collagène par les cellules, par rapport au contrôle négatif.

Les résultats obtenus sont montrés dans le tableau 1 ci-après.

**Tableau 1 - effet d'un extrait fermenté selon l'invention sur la production de collagène par des fibroblastes humains**

| | Rapport collagène / protéines Bradford | | % d'augmentation de la production de collagène par rapport au contrôle négatif | |
|---|---|---|---|---|
| | Matrice cellulaire | Milieu de culture | Matrice cellulaire | Milieu de culture |
| Extrait fermenté selon l'invention 5000 µg/ml | 0,198 | 0,185 | 29 | 0 |
| Extrait fermenté selon l'invention 1000 µg/ml | 0,212 | 0,209 | 38 | 13 |
| Extrait fermenté selon l'invention 500 µg/ml | 0,145 | 0,172 | 3 | 0 |
| Contrôle positif (TGF-β 10 ng/ml) | 0,233 | 0,168 | 51 | 0 |
| Contrôle négatif (milieu de culture) | 0,154 | 0,185 | 72 | 0 |

La figure 2 représente un graphe montrant le pourcentage d'augmentation de la production de collagène par les cellules de fibroblastes humains, induite par l'extrait fermenté selon l'invention aux différentes concentrations dans le milieu de culture des cellules, et par le facteur de croissance TGF-β, par rapport au milieu de culture non additionné.

Il ressort clairement des résultats obtenus que l'extrait fermenté de feuilles de *Viola alba* subsp. *dehnhardtii* conforme à l'invention augmente de manière significative la production de collagène par les fibroblastes humains, et ce dès la concentration de 1000 µg/ml dans le milieu de culture cellulaire.

Ces résultats démontrent la performance de l'extrait fermenté selon l'invention pour lutter contre le vieillissement cutané.

### D/ Action contre la formation de biofilm par Propionibacterium acnes et par Staphylococcus epidermitis

L'extrait de feuilles de *Viola alba* subsp. *dehnhardtii* fermenté obtenu à l'exemple A/ est testé pour sa capacité à lutter contre les biofilms de *Propionibacterium acnes* et contre les biofilms de *Staphylococcus epidermitis.*

Le but de cette étude est de déterminer si la capacité de ces bactéries à établir un biofilm, impliqué dans l'acné, peut être freinée, et la maturation de ce biofilm bloquée, par l'extrait fermenté selon l'invention. A cet effet, il est utilisé la méthode du Biofilm Ring Test® de la société Biofilm Control®. Cette méthode permet de déterminer un indice, appelé BFI (pour l'anglais BioFilm Index), qui est inversement proportionnel au niveau de production de biofilm (Dagnelie et al., 2018, Annales de Dermatologie et de Vénéréologie 145(12): S123).

Dans ces expériences, le pourcentage de présence du biofilm est déterminé initialement (à 0 h) et après respectivement 2h, 4h, 6h et 24h d'incubation des bactéries avec l'extrait selon l'invention.

Les organismes utilisés dans cette étude sont *Propionibacterium acnes* KPA 171202 et *Staphylococcus epidermidis* ATCC 12228.

Les protocoles mis en œuvre sont les suivants.

Concernant *Propionibacterium acnes,* un aliquot liquide de bactéries est préparé selon la méthode de Hayes et Markovic légèrement modifiée (Hayes et Markovic, 2002, Food Chem Toxicol. 40: 535-543). Des cellules de *Propionibacterium acnes* sont incubées dans un milieu pour perfusion de cœur et de cerveau (BHI) avec 1% de glucose pendant 48 h à 37°C et dans des conditions anaérobies. Le milieu est dilué pour obtenir environ 1,0x10⁸ UFC/ml.

Des cellules de *Staphylococcus epidermidis* sont incubées dans du bouillon trypticase soja (TSB) pendant 24h à 37 °C en conditions aérobies, puis le milieu est ajusté pour obtenir environ 1,0x10⁸ UFC/ml.

Les compositions suivantes sont préparées :
Témoin : des éprouvettes de 10 ml contenant 3 ml du bouillon de glucose de levure nutritif (NYG) pour *Propionibacterium acnes,* ou 3 ml du bouillon de nutriments pour *Staphylococcus epidermidis,* ont été stérilisées à l'autoclave pendant 12h à 150 °C. Le milieu dans chaque éprouvette a été refroidi puis ensemencé avec 50 µl de la suspension bactérienne de *Propionibacterium acnes* ou de *Staphylococcus epidermidis* contenant 1x10⁸ cellules/ml.
Compositions à base d'extrait selon l'invention : l'extrait selon l'invention est ajouté, respectivement à 2 % v/v (composition S2%) et à 4 % v/v (composition S4%), dans certaines des éprouvettes ci-dessus.

Le principe de la méthode Biofilm Ring Test® est décrit par Chavant et al. 1997, J Med Microbiol 60: 300-306. Il est basé sur l'addition à la solution bactérienne, contenant le cas échéant l'extrait à tester, de billes magnétiques de 5 ou 6 µm de diamètre. Le mélange de billes magnétiques et de solution bactérienne est ensemencé dans des puits, puis incubé pendant différentes durées comprises entre 0h et 24h. Les conditions d'aérobiose ou d'anaérobiose nécessaires au développement de biofilms bactériens pour chacune des souches testées sont respectées. Après la période d'incubation dans les puits, un produit de contraste est ajouté pour permettre la lecture dans le lecteur spécial Biofilm Reader®. Après la première lecture, les données collectées par le lecteur sont envoyées à l'ordinateur, interprétées et stockées par le logiciel spécial Biofilm Control Elements®. Les puits sont ensuite insérés dans le dispositif de magnétisation et magnétisés pendant 1 min. Les billes qui n'ont pas été retenues dans le biofilm éventuellement formé sont attirées vers le centre du puits, y formant un rond rouge. Au fur et à mesure de la formation du biofilm, les particules y sont immobilisées dans tout le volume du puits et le rond rouge disparait. Après l'aimantation, une seconde lecture est effectuée pour permettre la détection des billes magnétiques déposées sur la base du puits. Les données recueillies après cette deuxième lecture sont également transmises, interprétées et stockées dans l'ordinateur. À partir de la comparaison des images obtenues après la première et la deuxième lecture pour chaque puits, une valeur appelée Indice de formation de biofilm (BFI) est calculée, ainsi que le pourcentage de présence de biofilm dans le puits.

La figure 3 montre des photographies des puits après différents temps d'incubation pour le témoin (« T ») et pour les deux concentrations d'extrait fermenté selon l'invention (« S2% » et « S4% ») pour les différents temps d'incubation, en a/ pour *Propionibacterium acnes* et en b/ pour *Staphylococcus epidermitis.*

Comme on peut l'observer, concernant *Propionibacterium acnes,* à 0h tous les échantillons montrent une absence de biofilm (anneau central bien visible sur les photographies) ; à partir de 2h d'incubation, le témoin produit du biofilm en quantité et ce jusqu'à 24h d'incubation (l'anneau central n'est plus visible, témoignant du fait que les particules ont été immobilisées dans le biofilm formé). Pour les échantillons contenant l'extrait selon l'invention (S2% et S4%) on observe une absence de biofilm à toutes les durées d'incubation testées.

Concernant *Staphylococcus epidermitis,* de 0h à 4h tous les échantillons montrent une absence de biofilm ; à partir de 6h d'incubation le témoin produit du biofilm en quantité, et ce jusqu'à 24h. Pour les échantillons contenant l'extrait selon l'invention (S2% et S4%) on observe l'absence de biofilm à toutes les durées d'incubation testées.

Pour tous les échantillons, le pourcentage de présence de biofilm après 24h d'incubation est calculé. Les résultats sont montrés sur la figure 4 pour *Propionibacterium acnes* et sur la figure 5 pour *Staphylococcus epidermitis.*

Comme on peut l'observer, le témoin montre que *Propionibacterium acnes* est biofilmogène à 100%, c'est-à-dire que le quorum sensing est maximum et produit du biofilm. L'échantillon selon l'invention S2% montre une diminution de la formation de biofilm de 55%, donc de plus de la moitié par rapport au témoin. L'échantillon selon l'invention S4% montre une diminution de la formation de biofilm de 85% c'est-à-dire que le quorum sensing n'a pas pu développer la maturation du biofilm.

Concernant *Staphylococcus epidermitis,* là encore le témoin montre que *Staphylococcus epidermitis est* biofilmogène à 100%, c'est-à-dire que le quorum sensing est maximum et produit du biofilm. L'échantillon selon l'invention S2% montre une diminution de la formation de biofilm de 46% par rapport au témoin. L'échantillon selon l'invention S4% montre une diminution de la formation de biofilm de 72%, c'est-à-dire que le quorum sensing n'a pas pu développer la maturation du biofilm.

L'ensemble de ces résultats démontre bien l'efficacité anti-biofilm de l'extrait fermenté selon l'invention sur les deux souches de bactéries testées, qui sont associées aux troubles de l'acné. En empêchant la production de biofilm, l'extrait selon l'invention prévient la formation de bactéries sessiles, et tant *Propionibacterium acnes* que *Staphylococcus epidermitis* restent en symbiose avec les kératinocytes de la peau et conservent leur activité commensale de protection de la barrière cutanée.

## Revendications

1. Extrait fermenté d'une partie aérienne de violette, obtenu par un procédé d'obtention comprenant des étapes de :
- préparation d'un extrait liquide par extraction aqueuse de ladite partie aérienne de violette,
- préparation d'une solution dite de fermentation par mélange dudit extrait liquide avec un sucre fermentescible,
**caractérisé en ce que** ledit procédé comporte des étapes de :
- ensemencement de ladite solution de fermentation avec un mélange de microorganismes contenant au moins une espèce de bactéries acétiques et au moins une espèce de levure, ledit mélange étant essentiellement dépourvu de bactéries lactiques et de levures fermentant le lactose,
- incubation à une température inférieure ou égale à 30 °C pendant au moins 10 jours,
- et récupération de l'extrait fermenté liquide obtenu.

2. Extrait selon la revendication 1, selon lequel ledit procédé est mis en œuvre à partir de violette de l'espèce *Viola alba,* de préférence de la sous-espèce *Viola alba* subsp. *dehnhardtii.*

3. Extrait selon l'une quelconque des revendications 1 à 2, selon lequel ledit procédé est mis en œuvre à partir d'une partie aérienne de violette contenant des feuilles de violette, et préférentiellement essentiellement constituée de feuilles de violette.

4. Extrait selon l'une quelconque des revendications 1 à 3, selon lequel, dans ledit procédé d'obtention, l'extraction aqueuse est réalisée à une température comprise entre 50 et 95 °C, de préférence entre 80 et 95 °C.

5. Extrait selon l'une quelconque des revendications 1 à 4, selon lequel, dans ledit procédé d'obtention, ledit sucre fermentescible est le saccharose.

6. Extrait selon l'une quelconque des revendications 1 à 5, selon lequel, dans ledit procédé d'obtention, ledit mélange de microorganismes contient :
- au moins une espèce de bactéries du genre *Acetobacter* ou du genre *Gluconobacter,*
- et/ou au moins une levure de la famille des *Saccharomycetaceae* ou de la famille des *Schizosaccharomycetaceae.*

7. Extrait selon l'une quelconque des revendications 1 à 6, selon lequel, dans ledit procédé d'obtention, ledit mélange de microorganismes est supporté sur support solide, de préférence sur un support solide à base d'hémicellulose.

8. Extrait selon l'une quelconque des revendications 1 à 7, présentant un pH inférieur ou égal à 3 et un degré Brix inférieur ou égal à 2.

9. Procédé d'obtention d'un extrait fermenté d'une partie aérienne de violette selon l'une quelconque des revendications 1 à 8, **caractérisé en ce qu'**il comprend des étapes de :
- préparation d'un extrait liquide par extraction aqueuse de ladite patrie aérienne de violette,
- préparation d'une solution dite de fermentation par mélange dudit extrait liquide avec un sucre fermentescible,
- ensemencement de ladite solution de fermentation avec un mélange de microorganismes contenant au moins une espèce de bactéries acétiques et au moins une espèce de levure, ledit mélange étant essentiellement dépourvu de bactéries lactiques et de levures fermentant le lactose,
- incubation à une température inférieure ou égale à 30 °C pendant au moins 10 jours,
- et récupération de l'extrait fermenté liquide obtenu.

10. Composition contenant un extrait fermenté d'une partie aérienne de violette selon l'une quelconque des revendications 1 à 8, dans un véhicule physiologiquement compatible.

11. Procédé de traitement cosmétique non-thérapeutique de la peau, des muqueuses et/ou des cheveux d'un individu, **caractérisé en ce qu'**il comprend l'administration audit individu d'une composition selon la revendication 10.

12. Extrait fermenté d'une partie aérienne de violette selon l'une quelconque des revendications 1 à 8, pour son utilisation pour le traitement de l'acné et/ou des cicatrices.

## Patentansprüche

1. Fermentierter Extrakt eines oberirdischen Veilchenabschnitts, gewonnen durch ein Gewinnungsverfahren, das die folgenden Schritte umfasst:
- Herstellen eines flüssigen Extrakts durch wässrige Extraktion des oberirdischen Veilchenabschnitts,
- Herstellen einer so genannten Fermentationslösung durch Mischen des flüssigen Extrakts mit einem fermentierbaren Zucker,
**dadurch gekennzeichnet, dass** das Verfahren die folgenden Schritte umfasst:
- Beimpfen der Fermentationslösung mit einem Mikroorganismengemisch, das mindestens eine Essigsäurebakterienart und mindestens eine Hefeart enthält, wobei das Gemisch im Wesentlichen frei von Milchsäurebakterien und von Laktose fermentierenden Hefen ist,
- Inkubieren bei einer Temperatur von unter oder gleich 30°C während mindestens 10 Tagen,
- und Rückgewinnen des gewonnenen flüssigen fermentierten Extrakts.

2. Extrakt nach Anspruch 1, wobei das Verfahren auf der Basis von Veilchen der Art *Viola alba,* vorzugsweise der Unterart *Viola alba* subsp. *Dehnhardtii* durchgeführt wird.

3. Extrakt nach einem der Ansprüche 1 bis 2, wobei das Verfahren auf der Basis eines oberirdischen Veilchenabschnitts durchgeführt wird, der Veilchenblätter enthält und vorzugsweise im Wesentlichen aus Veilchenblättern besteht.

4. Extrakt nach einem der Ansprüche 1 bis 3, wobei bei dem Gewinnungsverfahren die wässrige Extraktion bei einer Temperatur zwischen 50 und 95°C, vorzugsweise zwischen 80 und 95°C, durchgeführt wird.

5. Extrakt nach einem der Ansprüche 1 bis 4, wobei bei dem Gewinnungsverfahren der fermentierbare Zucker die Saccharose ist.

6. Extrakt nach einem der Ansprüche 1 bis 5, wobei bei dem Gewinnungsverfahren das Mikroorganismengemisch enthält:
- mindestens eine Bakterienart der Gattung *Acetobacter* oder der Gattung *Gluconobacter,*
- und/oder mindestens eine Hefe aus der Familie der *Saccharomycetaceae* oder der Familie der *Schizosaccharomycetaceae.*

7. Extrakt nach einem der Ansprüche 1 bis 6, wobei bei dem Gewinnungsverfahren das Mikroorganismengemisch auf einem festen Träger, vorzugsweise auf einem festen Träger auf der Basis von Hemicellulose, getragen wird.

8. Extrakt nach einem der Ansprüche 1 bis 7, aufweisend einen pH von unter oder gleich 3 und einen Brix-Grad von unter oder gleich 2.

9. Gewinnungsverfahren eines fermentierten Extrakts eines oberirdischen Veilchenabschnitts nach einem der Ansprüche 1 bis 8, **dadurch gekennzeichnet, dass** es folgende Schritte umfasst:
- Herstellen eines flüssigen Extrakts durch wässrige Extraktion des oberirdischen Veilchenabschnitts,
- Herstellen einer so genannten Fermentationslösung durch Mischen des flüssigen Extrakts mit einem fermentierbaren Zucker,
- Beimpfen der Fermentationslösung mit einem Mikroorganismengemisch, das mindestens eine Essigsäurebakterienart und mindestens eine Hefeart enthält, wobei das Gemisch im Wesentlichen frei von Milchsäurebakterien und von Laktose fermentierenden Hefen ist,
- Inkubieren bei einer Temperatur von unter oder gleich 30°C während mindestens 10 Tagen,
- und Rückgewinnen des erhaltenen flüssigen fermentierten Extrakts.

10. Zusammensetzung, die einen fermentierten Extrakt eines oberirdischen Veilchenabschnitts nach einem der Ansprüche 1 bis 8 in einem physiologisch kompatiblen Vehikel enthält.

11. Nicht therapeutisches kosmetisches Verfahren zur Behandlung der Haut, der Schleimhäute und/oder der Haare eines Individuums, **dadurch gekennzeichnet, dass** es die Verabreichung einer Zusammensetzung nach Anspruch 10 an das Individuum umfasst.

12. Fermentierter Extrakt eines oberirdischen Veilchenabschnitts nach einem der Ansprüche 1 bis 8 für seine Verwendung zur Behandlung der Akne und/oder der Narben.

## Claims

1. Fermented extract of a violet aerial part, obtained by an obtention method comprising steps of:
- preparing a liquid extract by aqueous extraction of said violet aerial part,
- preparing a so-called fermentation solution by mixing said liquid extract with a fermentable sugar,
**characterised in that** said method includes steps of:
- seeding said fermentation solution with a mixture of microorganisms containing at least one species of acetic bacteria and at least one species of yeast, said mixture being essentially devoid of lactic bacteria and of lactose-fermenting yeasts,
- incubating at a temperature less than or equal to 30°C for at least 10 days,
- and retrieving the liquid fermented extract obtained.

2. Extract according to claim 1, wherein said method is implemented using violet of the species *Viola alba,* preferably of the subspecies *Viola alba* subsp. *dehnhardtii.*

3. Extract according to any one of claims 1 to 2, wherein said method is implemented using a violet aerial part containing violet leaves, and preferably essentially consisting of violet leaves.

4. Extract according to any one of claims 1 to 3, wherein, in said obtention method, the aqueous extraction is carried out at a temperature between 50 and 95°C, preferably between 80 and 95°C.

5. Extract according to any one of claims 1 to 4, wherein, in said obtention method, said fermentable sugar is sucrose.

6. Extract according to any one of claims 1 to 5, wherein, in said obtention method, said mixture of microorganisms contains:
- at least one species of bacteria of the *Acetobacter* genus or of the *Gluconobacter* genus,
- and/or at least one yeast of the *Saccharomycetaceae* family or of the *Schizosaccharomycetaceae* family.

7. Extract according to any one of claims 1 to 6, wherein, in said obtention method, said mixture of microorganisms is supported on a solid substrate, preferably on a hemicellulose-based solid substrate.

8. Extract according to any one of claims 1 to 7, having a pH less than or equal to 3 and a Brix degree less than or equal to 2.

9. Method for obtaining a fermented extract of a violet aerial part according to any one of claims 1 to 8, **characterised in that** it comprises steps of:
- preparing a liquid extract by aqueous extraction of said violet aerial part,
- preparing a so-called fermentation solution by mixing said liquid extract with a fermentable sugar,
- seeding said fermentation solution with a mixture of microorganisms containing at least one species of acetic bacteria and at least one species of yeast, said mixture being essentially devoid of lactic bacteria and of lactose-fermenting yeasts,
- incubating at a temperature less than or equal to 30°C for at least 10 days,
- and retrieving the liquid fermented extract obtained.

10. Composition containing a fermented extract of a violet aerial part according to any one of claims 1 to 8, in a physiologically compatible vehicle.

11. Method for the non-therapeutic cosmetic treatment of an individual's skin, mucosa and/or hair, **characterised in that** it comprises administering a composition according to claim 10 to said individual.

12. Fermented extract of a violet aerial part according to any one of claims 1 to 8, for use for the treatment of acne and/or scars.
